# EUROPEAN PATENT APPLICATION

(11) **EP 4 728 965 A1**
(43) Date of publication of application: **22.04.2026**
(21) Application number: 24823410.6
(22) Date of filing: 12.06.2024
(51) Int. Cl.: A61B 3/10, G01N 21/17, G01N 21/21

(54) **OPTICAL TOMOGRAPHIC IMAGING DEVICE**

(30) Priority: 14.06.2023 JP 2023097533
(71) Applicant: Tomey Corporation, Nagoya-shi, Aichi 451-0051 (JP)
(72) Inventor: SUGIYAMA Satoshi, Nagoya-shi, Aichi 451-0051 (JP); YAMANARI Masahiro, Nagoya-shi, Aichi 451-0051 (JP); SUCHANDRA Banerjee, Nagoya-shi, Aichi 451-0051 (JP)
(74) Representative: Winter, Brandl - Partnerschaft mbB
(86) International application number: PCT/JP2024/021334
(87) International publication number: WO 2024/257795

(57) **Abstract**

An optical coherence tomographic device may be a polarization-sensitive optical coherence tomographic device. The optical coherence tomographic device may include: an image capturing unit configured to capture a tomographic image of a subject eye; a range specifying means for specifying a depth range of the tomographic image; and a processor. The tomographic image may include a first tomographic image captured by irradiating the subject eye with first polarized light, and a second tomographic image captured by irradiating the subject eye with second polarized light that has a polarization direction different from that of the first polarized light. The processor may be configured to perform an image generation process for generating at least one of an image indicating birefringence and an image indicating a state of fibers for the depth range of the tomographic image specified by the range specifying means based on the first tomographic image and the second tomographic image.

## Description

### TECHNICAL FIELD

The technology disclosed in the present specification relates to a polarization-sensitive optical coherence tomographic device.

### BACKGROUND ART

Optical coherence tomographic devices are widely used in ophthalmic instruments and the like as a method for acquiring tomographic images of biological tissues since they are noninvasive and noncontact. In recent years, polarization-sensitive optical coherence tomographic devices, which are a kind of multifunctional optical coherence tomographic device, have been developed. Birefringence, which alters the state of polarization, occurs in tissues where molecules or fibrous structures are aligned in a particular direction. Birefringence can be visualized using a polarization-sensitive optical coherence tomographic device. For example, Japanese Patent Application Publication No. 2016-57197 describes an example of polarization-sensitive optical coherence tomographic device.

### SUMMARY

### TECHNICAL PROBLEM

Using a polarization-sensitive optical coherence tomographic device, such as the one described in Japanese Patent Application Publication No. 2016-57197, enables visualization of various birefringence-related characteristics. Accordingly, a polarization-sensitive optical coherence tomographic device enables generation of images containing a lot of information that cannot be acquired from tomographic images showing cross sections of a subject eye (hereinafter also referred to as normal tomographic images), enabling multifaceted analysis of the condition of the subject eye. However, images generated using the information acquired by a polarization-sensitive optical coherence tomographic device are more complex than normal tomographic images, which may make it difficult to accurately interpret clinically useful information.

The present specification discloses a technology for generating simple and useful images from information acquired by a polarization-sensitive optical coherence tomographic device.

### SOLUTION TO TECHNICAL PROBLEM

In a first aspect of the technology disclosed herein, a polarization-sensitive optical coherence tomographic device may comprise: an image capturing unit configured to capture a tomographic image of a subject eye; a range specifying means for specifying a depth range of the tomographic image; and a processor. The tomographic image may comprise a first tomographic image captured by irradiating the subject eye with first polarized light, and a second tomographic image captured by irradiating the subject eye with second polarized light that has a polarization direction different from that of the first polarized light. The processor may be configured to perform an image generation process for generating at least one of an image indicating birefringence and an image indicating a state of fibers for the depth range of the tomographic image specified by the range specifying means based on the first tomographic image and the second tomographic image.

The above optical coherence tomographic device specifies a depth range and generates at least one of an image indicating birefringence and an image indicating a state of fibers for the specified range, thereby generating image(s) for the depth range specified by a user. Since information from unnecessary depth ranges is eliminated by the user, amount of information can be reduced. This improves image visibility and allows for easy and accurate evaluation of the subject eye's condition.

### BRIEF DESCRIPTION OF DRAWINGS

[FIG. 1] a diagram schematically illustrating a configuration of an optical system of an optical coherence tomographic device according to first to seventh embodiments.
[FIG. 2] a block diagram illustrating a control system of the optical coherence tomographic device according to the first to seventh embodiments.
[FIG. 3] a block diagram illustrating a configuration of a sampling trigger/clock generator.
[FIG. 4] a flowchart illustrating an example of a process for generating a birefringence intensity map for a desired depth range of a subject eye, according to the first embodiment.
[FIG. 5] (a) is a tomographic image indicating birefringence, (b) is the image of (a) with boundaries between layers superimposed thereon, and (c) is an image indicating a birefringence intensity map only for a selected depth range of a subject eye.
[FIG. 6] a flowchart illustrating an example of a process for generating a fiber orientation map for a desired depth range of the subject eye, according to the second embodiment.
[FIG. 7] (a) is a tomographic image indicating fiber orientations with boundaries between layers superimposed thereon and (b) is an image indicating a fiber orientation map only for a selected depth range r1 (including the optical nerve fiber layer (NFL) and Henle's fiber layer) of the subject eye.
[FIG. 8] (a) is a tomographic image indicating fiber orientations with boundaries between layers superimposed thereon and (b) is an image indicating a fiber orientation map only for a selected depth range r2 (including the sclera) of the subject eye.
[FIG. 9] (a) is a tomographic image indicating fiber orientations with boundaries between layers superimposed thereon and (b) is an image of a fiber orientation map with streamlines superimposed thereon for the selected depth range r1 of the subject eye.
[FIG. 10] (a) is a tomographic image indicating fiber orientations with boundaries between layers superimposed thereon and (b) is an image of a fiber orientation map with streamlines superimposed thereon for the selected depth range r2 of the subject eye.
[FIG. 11] a flowchart illustrating an example of a process for displaying a high-birefringence regions over streamlines for a desired depth region of the subject eye, according to the fourth embodiment.
[FIG. 12] (a) is a tomographic image indicating fiber orientations with boundaries between layers superimposed thereon and (b) is an image indicating streamlines with high-birefringence regions superimposed thereon for the selected depth range r1 of the subject eye.
[FIG. 13] (a) is a tomographic image indicating fiber orientations with boundaries between layers superimposed thereon and (b) is an image indicating streamlines with high-birefringence regions superimposed thereon for the selected depth range r2 of the subject eye.
[FIG. 14] a flowchart illustrating an example of a process for generating a high-birefringence region thickness map for a desired depth range of the subject eye, according to the fifth embodiment.
[FIG. 15] (a) is a tomographic image indicating birefringence with boundaries between layers superimposed thereon, (b) is an image indicating high-birefringence regions for a selected depth region of the subject eye; and (c) is an image indicating a high-birefringence region thickness map only for the selected depth range of the subject eye.
[FIG. 16] (a) is a tomographic image indicating birefringence with boundaries between layers superimposed thereon, (b) is an image indicating high-birefringence regions for a selected depth region of the subject eye; and (c) is an image indicating a high-birefringence layer count map only for the selected depth range of the subject eye.
[FIG. 17] a flowchart illustrating an example of a process for generating a relative angle map for a desired depth range of the subject eye, according to the seventh embodiment.
[FIG. 18] a tomographic image indicating birefringence.
[Fig. 19] a tomographic image indicating fiber orientations in a relevant area XIX in FIG. 18.
[FIG. 20] a tomographic image indicating fiber orientations in a relevant area XX in FIG. 18.
[FIG. 21] an image indicating a relative angle map for a selected depth range of the subject eye.

### DESCRIPTION OF EMBODIMENTS

Some of the features characteristic to below-described embodiments will herein be listed. It should be noted that the respective technical elements are independent of one another, and are useful solely or in combinations. The combinations thereof are not limited to those described in the claims as originally filed.

In a second aspect of the technology disclosed in the present specification according to the first aspect, the optical coherence tomographic device may further comprise a display unit configured to display the image indicating the state of fibers. The display unit may be configured to display lines indicating fiber directions together with the image indicating the state of fibers such that the lines indicating fiber directions are superimposed on corresponding portions of the image indicating the state of fibers. In images indicating a state of fibers, fibers are generally colored in different colors according to their orientations, but it is difficult to read the fiber orientations solely from the color differences. By displaying lines indicating fiber orientations over an image indicating a state of fibers, the fiber directions can be easily recognized.

In a third aspect of the technology disclosed in the present specification according to the second aspect, the display unit may be further configured to display an image indicating a region where a birefringence intensity is greater than a predetermined value so as to be superimposed on a corresponding portion of the image indicating the state of fibers. With this configuration, a relationship between the fiber orientations and the region with high birefringence intensity can be easily recognized. It is useful to evaluate the birefringence intensity and fiber orientation separately, but it is also important to evaluate them comprehensively. By displaying the image indicating a region where a birefringence intensity is greater than the predetermined value over the image indicating the state of fibers, the birefringence intensity and fiber orientation can be easily evaluated comprehensively.

In a fourth aspect of the technology disclosed in the present specification according to the first aspect, the optical coherence tomographic device may further comprise a display unit configured to display the image indicating birefringence. The display unit may be configured to display a thickness map indicating thicknesses of regions where birefringence is greater than a predetermined value. With this configuration, thicknesses of regions with high birefringence can be easily grasped. For example, an intensity map indicating birefringence intensities provides the locations of regions with high birefringence, while grasping the thicknesses of the regions with high birefringence requires inspection of each tomographic image. By displaying a thickness map indicating thicknesses of regions where birefringence is greater than the predetermined value, the thicknesses of the regions with high birefringence can be readily grasped.

In a fifth aspect of the technology disclosed in the present specification according to the first aspect, the optical coherence tomographic device may further comprise a display unit configured to display the image indicating birefringence. The processor may be configured to further perform: a layer segmentation process for segmenting layers of the subject eye; and a determination process for determining, for each of the layers segmented in the layer segmentation process, whether birefringence is greater than a predetermined value or not. The display unit may be configured to display a layer count map indicating a number of layers determined to have birefringence greater than the predetermined value in the determination process. With this configuration, the number of layers having birefringence greater than the predetermined value can be easily grasped.

In a sixth aspect of the technology disclosed in the present specification according to the first aspect, the processor may be configured to further perform: a layer segmentation process for segmenting layers of the subject eye; a first calculation process for calculating, for each of the layers segmented in the layer segmentation process, a relative angle between the layer and an adjacent layer; and a second calculation process for calculating a sum of the relative angles calculated in the first calculation process for the depth range of the tomographic image specified by the range specifying means. The optical coherence tomographic device may further comprise a display unit configured to display an angle map indicating the sum of the relative angles calculated in the second calculation process. With this configuration, relative angles in the subject eye can be easily grasped. Information on how much fiber orientations differ between adjacent layers has clinical significance; for example, it can indicate whether the subject eye is resistant to stress associated with axial elongation. By displaying the relative angle map, the distribution of the sum of relative angles can be easily recognized.

### EMBODIMENTS

### (First Embodiment)

Referring to the drawings, an optical coherence tomographic device according to the present embodiment will be described. The optical coherence tomographic device according to the present embodiment is a polarization-sensitive OCT (PS-OCT) that is capable of capturing polarization characteristics of a subject to be examined by a Fourier domain method of a wavelength sweeping type using a wavelength sweeping light source (swept-source optical coherence tomography: SS-OCT).

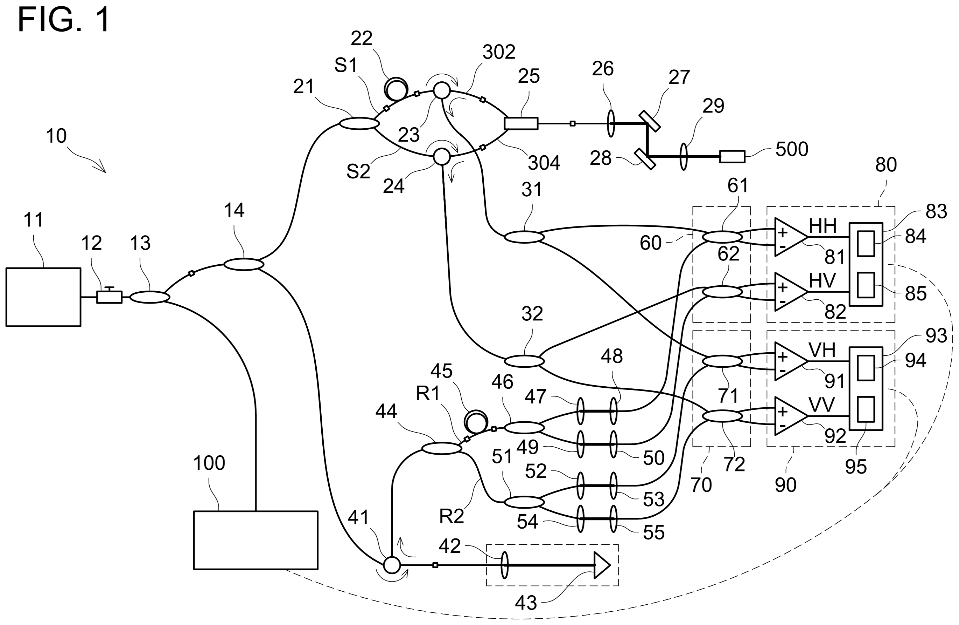

As illustrated in FIG. 1, the optical coherence tomographic device according to the present embodiment comprises a light source 11; a measurement light generator (21 to 29, 31, 32) that generates measurement light from light outputted from the light source 11; a reference light generator (41 to 46, 51) that generates reference light from the light outputted from the light source 11; interference light generators 60, 70 that combine reflected light from a subject eye 500 generated in the measurement light generator with the reference light generated in the reference light generator to generate interference light; and interference light detectors 80, 90 that detect the interference light generated in the interference light generators 60, 70.

### (Light Source)

The light source 11 is a light source of a wavelength sweeping type, and the wavelength (wavenumber) of output light varies within a predetermined cycle. Since the wavelength of light with which the subject eye 500 is irradiated varies (swept), an intensity distribution of light reflected from respective portions of the subject eye 500 along a depth direction can be obtained by subjecting signals obtained from interference light, which is a combination of the reflected light from the subject eye 500 and the reference light, to Fourier analysis.

A polarization control device 12 and a fiber coupler 13 are connected to the light source 11, and a PMFC (polarization maintaining fiber coupler) 14 and a sampling trigger/clock generator 100 are connected to the fiber coupler 13. Therefore, the light outputted from the light source 11 is inputted to the PMFC 14 and the sampling trigger/clock generator 100 through the polarization control device 12 and the fiber coupler 13. The sampling trigger/clock generator 100 generates a sampling trigger and a sampling clock for each of signal processors 83 and 93 (which will be described later) by using the light from the light source 11.

### (Measurement Light Generator)

The measurement light generator (21 to 29, 31, 32) comprises a PMFC 21 connected to the PMFC 14; two measurement light paths S1 and S2 branching off from the PMFC 21; a polarization beam combiner/splitter 25 connecting the two measurement light paths S1 and S2; a collimator lens 26 connected to the polarization beam combiner/splitter 25; galvanometer mirrors 27 and 28; and a lens 29. An optical path length difference generator 22 and a circulator 23 are disposed on the measurement light path S1. Only a circulator 24 is disposed on the measurement light path S2. Therefore, an optical path length difference ΔL between the measurement light path S1 and the measurement light path S2 is generated by the optical path length difference generator 22. The optical path length difference ΔL may be set to be longer than a depthwise measurement range of the subject eye 500. This prevents interference light with different optical path length differences from overlapping each other. As the optical path length difference generator 22, for example, an optical fiber may be used or an optical system such as a mirror, a prism, etc. may be used. In the present embodiment, a PM fiber with a length of one meter is used as the optical path length difference generator 22. The measurement light generator further comprises PMFCs 31, 32. The PMFC 31 is connected to the circulator 23. The PMFC 32 is connected to the circulator 24.

One of light (i.e., measurement light) split by the PMFC 14 is inputted to the measurement light generator (21 to 29, 31, 32). The PMFC 21 splits the measurement light inputted from the PMFC 14 into first measurement light and second measurement light. The first measurement light split by the PMFC 21 is guided to the measurement light path S1, and the second measurement light split by the PMFC 21 is guided to the measurement light path S2. The first measurement light guided to the measurement light path S1 is inputted to the polarization beam combiner/splitter 25 through the optical path length difference generator 22 and the circulator 23. The second measurement light guided to the measurement light path S2 is inputted to the polarization beam combiner/splitter 25 through the circulator 24. A PM fiber 304 is connected to the polarization beam combiner/splitter 25 such that the PM fiber 304 is circumferentially turned by 90 degrees relative to a PM fiber 302. For this reason, the second measurement light inputted to the polarization beam combiner/splitter 25 has a polarization component orthogonal to the first measurement light. Since the optical path length difference generator 22 is disposed on the measurement light path S1, the first measurement light is delayed relative to the second measurement light by a distance corresponding to the optical path length difference generator 22 (that is, the optical path length difference ΔL is generated). The polarization beam combiner/splitter 25 superimposes the inputted first measurement light and second measurement light. The light outputted from the polarization beam combiner/splitter 25 (superimposed light of the first measurement light and the second measurement light) passes through the collimator lens 26, the galvanometer mirrors 27 and 28, and the lens 29 and is then irradiated onto the subject eye 500. The light irradiated onto the subject eye 500 is scanned along an x-y direction by the galvanometer mirrors 27 and 28.

The light irradiated onto the subject eye 500 is reflected by the subject eye 500. The reflected light by the subject eye 500 scatters at the surface of the subject eye 500 and the inside thereof. The reflected light from the subject eye 500 passes through, in the reverse order to the incidence path, the lens 29, the galvanometer mirrors 28, 27, and the collimator lens 26, and is then inputted to the polarization beam combiner/splitter 25. The polarization beam combiner/splitter 25 splits the inputted reflected light into two polarization components that are orthogonal to each other. These are termed horizontal polarization reflected light (horizontal polarization component) and vertical polarization reflected light (vertical polarization component), for convenience sake. The horizontal polarization reflected light is guided to the measurement light path S1, and the vertical polarization reflected light is guided to the measurement light path S2.

The optical path of the horizontal polarization reflected light is changed by the circulator 23, and the horizontal polarization reflected light is inputted to the PMFC 31. The PMFC 31 splits the inputted horizontal polarization reflected light so that it is inputted to each of PMFCs 61, 71. Therefore, the horizontal polarization reflected light inputted to each of the PMFCs 61, 71 contains a reflected light component based on the first measurement light and a reflected light component based on the second measurement light. The optical path of the vertical polarization reflected light is changed by the circulator 24, and the vertical polarization reflected light is inputted to the PMFC 32. The PMFC 32 splits the inputted vertical polarization reflected light so that it is inputted to each of PMFCs 62, 72. Therefore, the vertical polarization reflected light inputted to each of the PMFCs 62, 72 contains a reflected light component based on the first measurement light and a reflected light component based on the second measurement light.

### (Reference Light Generator)

The reference light generator (41 to 46, 51) comprises a circulator 41 connected to the PMFC 14; a reference delay line (42, 43) connected to the circulator 41; a PMFC 44 connected to the circulator 41; two reference light paths R1 and R2 branching off from the PMFC 44; a PMFC 46 connected to the reference light path R1; and a PMFC 51 connected to the reference light path R2. An optical path length difference generator 45 is disposed on the reference light path R1. No optical path length difference generator is disposed on the reference light path R2. Therefore, an optical path length difference ΔL' between the reference light path R1 and the reference light path R2 is generated by the optical path length difference generator 45. For example, an optical fiber is used as the optical path length difference generator 45. The optical path length difference ΔL' of the optical path length difference generator 45 may be the same as the optical path length difference ΔL of the optical path length difference generator 22. If the optical path length differences ΔL and ΔL' are the same, depthwise positions of a plurality of interference light (described later) in the subject eye 500 coincide with each other. That is, it is unnecessary to align a plurality of acquired tomographic images.

The other of light split by the PMFC 14 (i.e., reference light) is inputted to the reference light generator (41 to 46, 51). The reference light inputted from the PMFC 14 is inputted to the reference delay line (42, 43) through the circulator 41. The reference delay line (42, 43) includes a collimator lens 42 and a reference mirror 43. The reference light inputted to the reference delay line (42, 43) is irradiated to the reference mirror 43 through the collimator lens 42. The reference light reflected by the reference mirror 43 is inputted to the circulator 41 through the collimator lens 42. The reference mirror 43 is movable in directions to approach and separate from the collimator lens 42. In the present embodiment, the position of the reference mirror 43 is adjusted before the start of measurement so that a signal from the subject eye 500 will be within an OCT depthwise measurable range.

The optical path of the reference light reflected by the reference mirror 43 is changed by the circulator 41, and the reference light reflected by the reference mirror 43 is inputted to the PMFC 44. The PMFC 44 splits the inputted reference light into first reference light and second reference light. The first reference light is guided to the reference light path R1, and the second reference light is guided to the reference light path R2. The first reference light is inputted to the PMFC 46 through the optical path length difference generator 45. The reference light inputted to the PMFC 46 is split into first split reference light and second split reference light. The first split reference light is inputted to the PMFC 61 through a collimator lens 47 and a lens 48. The second split reference light is inputted to the PMFC 62 through a collimator lens 49 and a lens 50. The second reference light is inputted to the PMFC 51 and then is split into third split reference light and fourth split reference light. The third split reference light is inputted to the PMFC 71 through a collimator lens 52 and a lens 53. The fourth split reference light is inputted to the PMFC 72 through a collimator lens 54 and a lens 55.

### (Interference Light Generator)

The interference light generators 60, 70 include a first interference light generator 60 and a second interference light generator 70. The first interference light generator 60 includes the PMFCs 61 and 62. As described, the horizontal polarization reflected light from the measurement light generator and the first split reference light (light having the optical path length difference ΔL') from the reference light generator are inputted to the PMFC 61. Here, the horizontal polarization reflected light contains a reflected light component (light having the optical path length difference ΔL) based on the first measurement light and a reflected light component (light that does not have the optical path length difference ΔL) based on the second measurement light. Therefore, in the PMFC 61, the first split reference light is combined with the reflected light component (light having the optical path length difference ΔL) based on the first measurement light which is among the horizontal polarization reflected light, as a result of which first interference light (horizontal polarization component) is generated.

The vertical polarization reflected light from the measurement light generator and the second split reference light (light having the optical path length difference ΔL') from the reference light generator are inputted to the PMFC 62. Here, the vertical polarization reflected light contains a reflected light component (light having the optical path length difference ΔL) based on the first measurement light and a reflected light component (light that does not have the optical path length difference ΔL) based on the second measurement light. Therefore, in the PMFC 62, the second split reference light is combined with the reflected light component (light having the optical path length difference ΔL) based on the first measurement light which is among the vertical polarization reflected light, as a result of which second interference light (vertical polarization component) is generated.

The second interference light generator 70 includes the PMFCs 71 and 72. As described, the horizontal polarization reflected light from the measurement light generator and the third split reference light (light that does not have the optical path length difference ΔL') from the reference light generator are inputted to the PMFC 71. Therefore, in the PMFC 71, the third split reference light is combined with a reflected light component (light that does not have the optical path length difference ΔL) based on the second measurement light which is among the horizontal polarization reflected light, as a result of which third interference light (horizontal polarization component) is generated.

The vertical polarization reflected light from the measurement light generator and the fourth split reference light (light that does not have the optical path length difference ΔL') from the reference light generator are inputted to the PMFC 72. Therefore, in the PMFC 72, the fourth split reference light is combined with the reflected light component (light that does not have the optical path length difference ΔL) based on the second measurement light which is among the vertical polarization reflected light, as a result of which fourth interference light (vertical polarization component) is generated. The first interference light and the second interference light correspond to the measurement light that has passed through the measurement light path S1, and the third interference light and the fourth interference light correspond to the measurement light that has passed through the measurement light path S2.

### (Interference Light Detectors)

The interference light detectors 80, 90 include a first interference light detector 80 configured to detect the interference light (the first interference light and the second interference light) generated in the first interference light generator 60, and a second interference light detector 90 configured to detect the interference light (the third interference light and the fourth interference light) generated in the second interference light generator 70.

The first interference light detector 80 comprises balanced light detectors 81 and 82 (which may simply be termed detectors 81, 82 hereinbelow), and a signal processor 83 connected to the detectors 81 and 82. The PMFC 61 is connected to the detector 81, and the signal processor 83 is connected to an output terminal of the detector 81. The PMFC 61 splits the first interference light into two interference light that have phases different from each other by 180 degrees, and inputs the two interference light to the detector 81. The detector 81 performs differential amplification processing and noise reduction processing to the two interference light having phases different from each other by 180 degrees inputted from the PMFC 61 so as to convert them to an electric signal (first interference signal), and outputs the first interference signal to the signal processor 83. That is, the first interference signal is an interference signal HH between the reference light and the horizontal polarization reflected light from the subject eye 500 based on the horizontal polarization measurement light. Similarly, the PMFC 62 is connected to the detector 82, and the signal processor 83 is connected to an output terminal of the detector 82. The PMFC 62 splits the second interference light into two interference light that have phases different from each other by 180 degrees, and inputs the two interference light to the detector 82. The detector 82 performs differential amplification processing and noise reduction processing to the two interference light having phases different from each other by 180 degrees so as to convert them to an electric signal (second interference signal), and outputs the second interference signal to the signal processor 83. That is, the second interference signal is an interference signal HV between the reference light and the vertical polarization reflected light from the subject eye 500 based on the horizontal polarization measurement light.

The signal processor 83 comprises a first signal processing unit 84 to which the first interference signal is inputted, and a second signal processing unit 85 to which the second interference signal is inputted. The first signal processing unit 84 is configured to sample the first interference signal based on a sampling trigger and a sampling clock inputted to the signal processor 83 from the sampling trigger/clock generator 100. The second signal processing unit 85 is configured to sample the second interference signal based on the sampling trigger and the sampling clock inputted to the signal processor 83 from the sampling trigger/clock generator 100. The first and second interference signals sampled in the first signal processing unit 84 and the second signal processing unit 85 are inputted to a processor 202 (which will be described later). A known data acquisition device (a so-called DAQ) may be used as the signal processor 83.

Similar to the first interference light detector 80, the second interference light detector 90 comprises balanced light detectors 91 and 92 (which may simply be termed detectors 91, 92 hereinbelow), and the signal processor 93 connected to the detectors 91 and 92. The PMFC 71 is connected to the detector 91, and the signal processor 93 is connected to an output terminal of the detector 91. The PMFC 71 splits the third interference light into two interference light that have phases different from each other by 180 degrees, and inputs the two interference light to the detector 91. The detector 91 performs differential amplification processing and noise reduction processing to the two interference light having phases different from each other by 180 degrees so as to convert them to an electric signal (third interference signal), and outputs the third interference signal to the signal processor 93. That is, the third interference signal is an interference signal VH between the reference light and the horizontal polarization reflected light from the subject eye 500 based on the vertical polarization measurement light. Similarly, the PMFC 72 is connected to the detector 92, and the signal processor 93 is connected to an output terminal of the detector 92. The PMFC 72 splits the fourth interference light into two interference light that have phases different from each other by 180 degrees, and inputs the two interference light to the detector 92. The detector 92 performs differential amplification processing and noise reduction processing to the two interference light having phases different from each other by 180 degrees so as to convert them to an electric signal (fourth interference signal), and outputs the fourth interference signal to the signal processor 93. That is, the fourth interference signal is an interference signal VV between the reference light and the vertical polarization reflected light from the subject eye 500 based on the vertical polarization measurement light.

The signal processor 93 comprises a third signal processing unit 94 to which the third interference signal is inputted, and a fourth signal processing unit 95 to which the fourth interference signal is inputted. The third signal processing unit 94 is configured to sample the third interference signal based on a sampling trigger and a sampling clock inputted to the signal processor 93 from the sampling trigger/clock generator 100. The fourth signal processing unit 95 is configured to sample the fourth interference signal based on the sampling trigger and the sampling clock inputted to the signal processor 93 from the sampling trigger/clock generator 100. The third and fourth interference signals sampled in the third signal processing unit 94 and the fourth signal processing unit 95 are inputted to the processor 202 (which will be described later). A known data acquisition device (a so-called DAQ) may also be used as the signal processor 93. According to the above configuration, it is possible to acquire the interference signals indicative of four polarization characteristics of the subject eye 500. In the present embodiment, the signal processors 83, 93, each of which comprises two signal processing units, are used, however, different configurations may be employed. For example, one signal processor comprising four signal processing units may be used, or four signal processors each comprising one signal processing unit may be used.

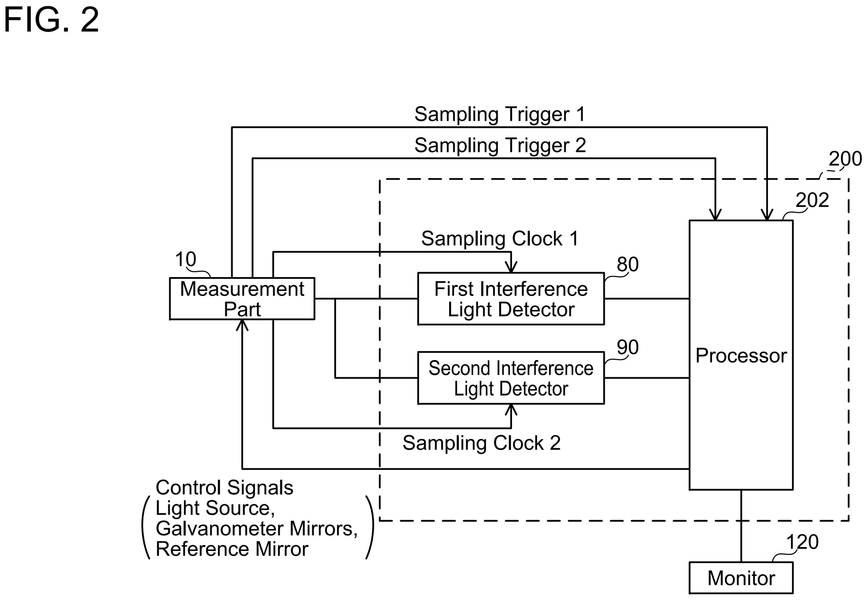

Next, the configuration of a control system of the optical coherence tomographic device according to the present embodiment will be described. As illustrated in FIG. 2, the optical coherence tomographic device is controlled by a calculation unit 200. The calculation unit 200 comprises the processor 202, the first interference light detector 80, and the second interference light detector 90. The first interference light detector 80, the second interference light detector 90, and the processor 202 are connected to a measurement unit 10. The processor 202 is configured to output a control signal to the measurement unit 10 to move an incidence position of the measurement light to the subject eye 500 by driving the galvanometer mirrors 27 and 28. The first interference light detector 80 acquires first sampling data with respect to the interference signals (the interference signal HH and the interference signal HV) inputted from the measurement unit 10 based on a sampling clock 1 inputted from the measurement unit 10 and by using a sampling trigger 1 as a trigger, and outputs the first sampling data to the processor 202. The processor 202 performs calculation processing such as Fourier transform, etc. to the first sampling data to generate an HH tomographic image and an HV tomographic image. The second interference light detector 90 acquires second sampling data with respect to the interference signals (the interference signal VH and the interference signal VV) inputted from the measurement unit 10 based on a sampling clock 2 inputted from the measurement unit 10 and by using a sampling trigger 2 as a trigger, and outputs the second sampling data to the processor 202. The processor 202 performs calculation processing such as Fourier transform, etc. to the second sampling data to generate a VH tomographic image and a VV tomographic image. The HH tomographic image, the VH tomographic image, the HV tomographic image, and the VV tomographic image are tomographic images at the same position. Thus, the processor 202 can create tomographic images with four polarization characteristics (HH, HV, VH, VV) that represent a Jones matrix of the subject eye 500.

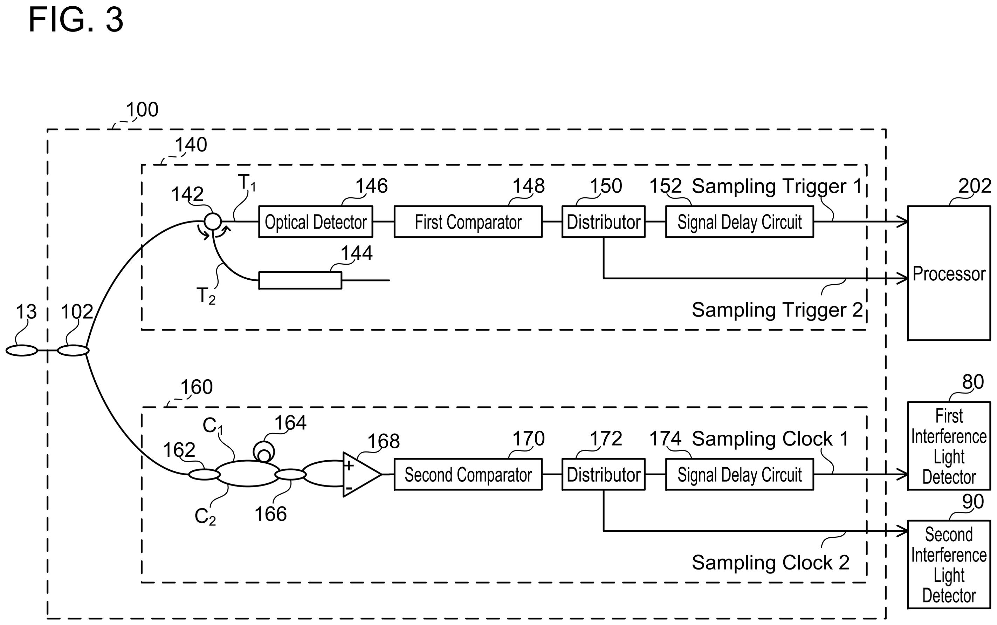

As illustrated in FIG. 3, the sampling trigger/clock generator 100 comprises a fiber coupler 102, a sampling trigger generator (140 to 152), and a sampling clock generator (160 to 172). The light from the light source 11 is inputted, through the fiber coupler 13 and the fiber coupler 102, to each of the sampling trigger generator 140 and the sampling clock generator 160.

### (Sampling Trigger Generator)

The sampling trigger generator 140 may generate a sampling trigger by using, for example, an FBG (fiber bragg grating) 144. As illustrated in FIG. 3, the FBG 144 reflects only a component of the light inputted from the light source 11 that has a specific wavelength, thereby generating a sampling trigger. The generated sampling trigger is inputted to a distributor 150. The distributor 150 distributes the sampling trigger into the sampling trigger 1 and the sampling trigger 2. The sampling trigger 1 is inputted, through a signal delay circuit 152, to the processor 202. The sampling trigger 2 is directly inputted to the processor 202. The sampling trigger 1 is a trigger signal for the interference signals (the first interference signal and the second interference signal) inputted from the first interference light detector 80 to the processor 202. The sampling trigger 2 is a trigger signal for the interference signals (the third interference signal and the fourth interference signal) inputted from the second interference light detector 90 to the processor 202. The signal delay circuit 152 is designed such that the sampling trigger 1 is delayed relative to the sampling trigger 2 by a time corresponding to the optical path length difference ΔL of the optical path length difference generator 22. This makes it possible to make a frequency at which the sampling of the interference signals inputted from the first interference light detector 80 is started equal to a frequency at which the sampling of the interference signals inputted from the second interference light detector 90 is started. Only the sampling trigger 1 may be generated. Since the optical path length difference ΔL is known, the sampling of the interference signals inputted from the second interference light detector 90 may be started such that the start time is delayed from the sampling trigger 1 by a time corresponding to the optical path length difference ΔL.

### (Sampling Clock Generator)

The sampling clock generator may be configured of a Mach-Zehnder interferometer, for example. As illustrated in FIG. 3, the sampling clock generator generates a sampling clock with the same frequency by using the Mach-Zehnder interferometer. The sampling clock generated by the Mach-Zehnder interferometer is inputted to a distributor 172. The distributor 172 distributes the sampling clock into the sampling clock 1 and the sampling clock 2. The sampling clock 1 is inputted, through a signal delay circuit 174, to the first interference light detector 80. The sampling clock 2 is directly inputted to the second interference light detector 90. The signal delay circuit 174 is designed to cause a delay by a time corresponding to the optical path length difference ΔL of the optical path length difference generator 22. This makes it possible to sample interference light with the delay corresponding to the optical path length difference generator 22 at the same timing. Thus, positional misalignment among a plurality of acquired tomographic images can be prevented. In the present embodiment, a Mach-Zehnder interferometer is used to generate the sampling clocks. Alternatively, a Michelson interferometer or an electric circuit may be used to generate the sampling clocks. Alternatively, the sampling clocks may be generated by using a light source including a sampling clock generator.

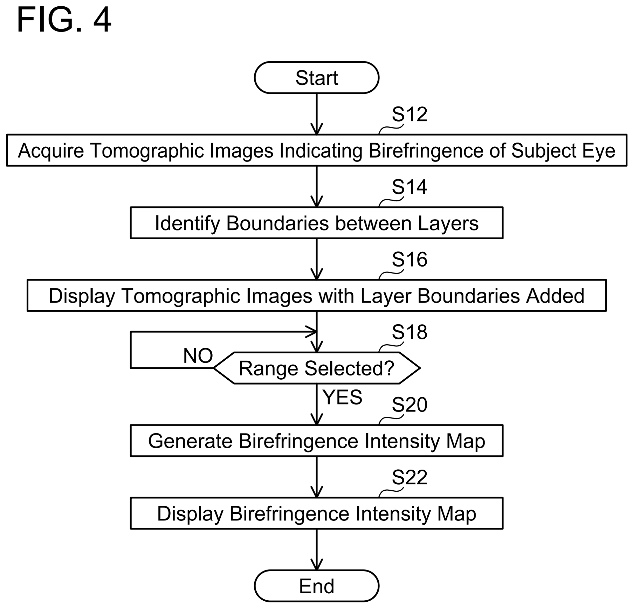

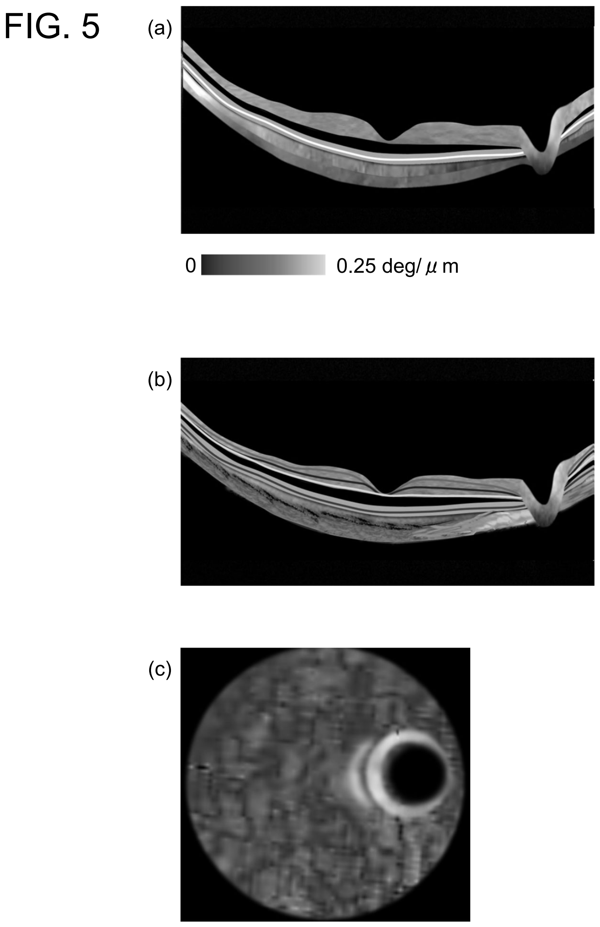

Referring now to FIGS. 4 and 5, a process for generating an image indicating birefringence intensities (hereinafter also referred to as a birefringence intensity map) for a desired depth range of the subject eye 500 is described. Capturing images of the subject eye 500 using an optical coherence tomographic device according to the present embodiment enables visualization of various birefringence-related characteristics. The depth range of the subject eye 500 that a user (e.g., a medical professional such as a physician) is interested in differs depending on the type of disease to be examined. If an image contains information outside the user-defined depth range, the image contains a large amount of information, which makes it difficult for the user to know what the user wants to know about the subject eye 500. In the following description, an image indicating birefringence (a birefringence intensity map) is generated only for a user-defined depth range. In particular, since a polarization-sensitive optical coherence tomographic device according to the present embodiment acquires a large amount of information, generating an image only for the user-defined depth range, excluding unnecessary ranges, improves visibility and usefulness of the image.

In the present embodiment, a process for superimposing and displaying a tomographic image of the retina of the subject eye 500 is described as an example. Tomographic images are not limited to those showing the retina of the subject eye 500. Tomographic images may be those showing another portion of the subject eye 500 other than the retina; for example, tomographic images may those showing the anterior segment.

As illustrated in FIG. 4, the processor 202 first acquires tomographic images indicating birefringence of the retina of the subject eye 500 (S12). The tomographic images indicating birefringence of the retina of the subject eye 500 are acquired by the following procedure. First, an examiner operates an operation member such as a joystick (not illustrated) to align the optical coherence tomographic device with the subject eye 500. That is, the processor 202 drives a position adjustment mechanism (not illustrated) in response to the examiner's operation on the operation member. Thereby, the positions of the optical coherence tomographic device in the x-y directions (vertical and lateral directions) and in a z direction (forward-backward direction) are adjusted relative to the subject eye 500. Then, the processor 202 captures tomographic images of the retina of the subject eye 500. In the present embodiment, this capture is performed according to a raster scan method. In the raster scan method, tomographic images are captured with the B scan direction set as a horizontal direction to the subject eye 500 and the C scan direction set as a vertical direction. Thereby, tomographic images of the entire retina of the subject eye 500 are acquired. The method of capturing tomographic images of the retina of the subject eye 500 is not limited to the raster scan method. Any method may be used as long as it can acquire tomographic images of the entire retina of the subject eye 500; for example, a radial scan method may be used. In the radial scan method, tomographic images are captured with the B scan direction set as a radial direction from the corneal apex of the subject eye 500 and the C scan direction set as a circumferential direction.

Since the optical coherence tomographic device according to the present embodiment is a polarization-sensitive optical coherence tomographic device as described above, it can simultaneously acquire a tomographic image captured by irradiating the subject eye 500 with vertically polarized light and a tomographic image captured by irradiating the subject eye 500 with horizontally polarized light. By using these two types of tomographic images, the processor 202 can generate not only tomographic images indicating tissues of the subject eye 500 with scattering intensities (so-called a normal tomographic image) but also tomographic images indicating birefringence within the subject eye 500. In the present embodiment, the tomographic images indicating birefringence are generated using four interference signals HH, HV, VH, and VV. The tomographic images indicating birefringence can be generated by a known method. For example, the tomographic images indicating birefringence can be acquired as follows. When capturing tomographic images, speckles arise due to interference among scattered light caused by microstructures smaller than the OCT resolution. The phase differences of the signals between polarizations of the generated speckles are displayed. Thereby, the tomographic images indicating birefringence can be acquired.

Then, the processor 202 identifies boundaries between adjacent tissues of the subject eye 500 (e.g., the boundary between the nerve fiber layer and the ganglion cell layer) (segments adjacent tissues of the subject eye 500) in each tomographic image (S14). Since this segmentation can be performed using a known method, for example, the one described in Japanese Patent Application Publication No. 2019-88957, detailed description thereof is omitted.

The processor 202 then superimposes the layer boundaries identified in step S14 onto a corresponding tomographic image acquired in step S12 and displays the result on the monitor 120 (S16). FIG. 5(a) illustrates a tomographic image acquired in step S12, and FIG. 5(b) illustrates the tomographic image of FIG. 5(a) with the boundaries identified in step S14 superimposed thereon. As illustrated in FIG. 5(b), by displaying the layer boundaries over the tomographic image, the user can easily and visually recognize each layer of the subject eye 500.

Then, the processor 202 determines whether a depth range has been selected (S18). Specifically, the user inputs a particular (desired) depth range in the tomographic image displayed on the monitor 120 in step S16 by using an input means (not illustrated) such as a mouse. For example, the user specifies one or more of the layers for which boundaries were identified in step S14. Thereby, the specified one or more layers are selected. Alternatively, when the user specifies a particular layer, a predetermined depth range extending from the front surface and/or the back surface of the specified layer may be selected. Alternatively, the user may input the name of a layer to be selected (e.g., nerve fiber layer (NFL)) using an input means (not illustrated) such as a keyboard, and the processor 202 may select one or more layers corresponding to the input layer. If a depth range is not selected (NO in step S18), the processor 202 waits until the user selects a depth range.

Once a depth range is selected (YES in step S18), the processor 202 generates a birefringence intensity map for the depth range selected in step S18 (S20). The birefringence intensity map is an image (en-face image) obtained by calculating, for each A scan, the maximum, minimum, or average value of birefringence intensity in the depth direction for three-dimensional data and compressing the three-dimensional data into a two-dimensional frontal image. The processor 202 generates the en-face image using only the tomographic images (tomographic information) within the depth range selected in step S18. As a result, a birefringence intensity map for only the depth range selected in step S18 is generated.

Then, the processor 202 displays the birefringence intensity map generated in step S20 on the monitor 120 (S22). As illustrated in FIG. 5(c), the birefringence intensity map which was calculated using tomographic information only from the one or more layers selected in step S18 is displayed on the monitor 120. The birefringence intensity map includes information only from the one or more layers selected in step S18 and does not include information from the other layers. Information on birefringence in unnecessary ranges is excluded and only the information on birefringence (the birefringence intensity map) in the desired range is displayed, thereby reducing unnecessary information and improving visibility of the image. It is known that fiber density correlates with birefringence. By checking the birefringence intensity distribution in the subject eye 500, the fiber density within the subject eye 500 can be indirectly evaluated.

### (Second Embodiment)

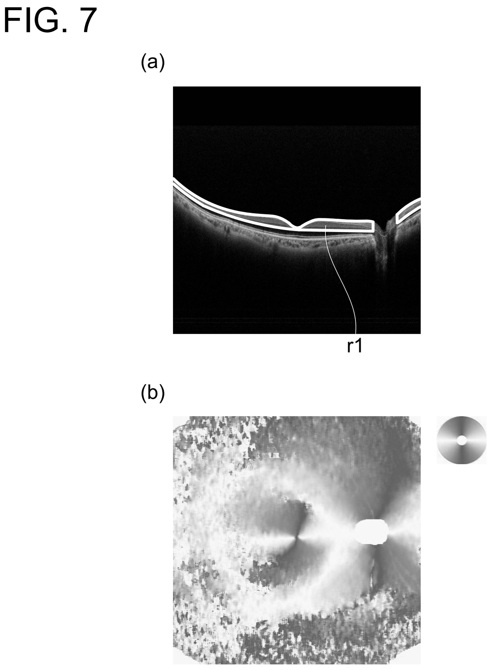

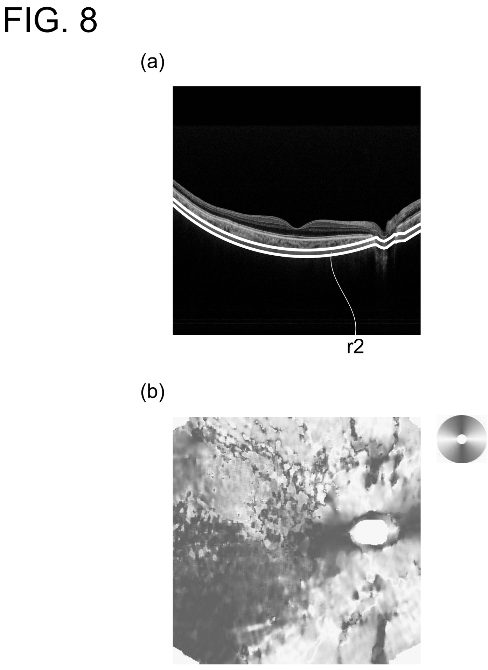

In the first embodiment described above, the processor 202 generates a birefringence intensity map for a desired depth range of the subject eye 500, but a different birefringence-related image may be generated for a desired depth range. For example, the processor 202 may generate an image indicating a state of fibrous tissues (hereinafter also referred to as a fiber orientation map) for a desired depth range of the subject eye 500. In the present embodiment, in the fiber orientation map, portions where the fiber orientation is horizontal is colored in a first color, portions where the fiber orientation is vertical is colored in a second color different from the first color, and intermediate portions are colored in a gradient between the first color and the second color (see FIGS. 7(b) and 8(b)). This allows for visual recognition of the fiber orientations based on the color differences.

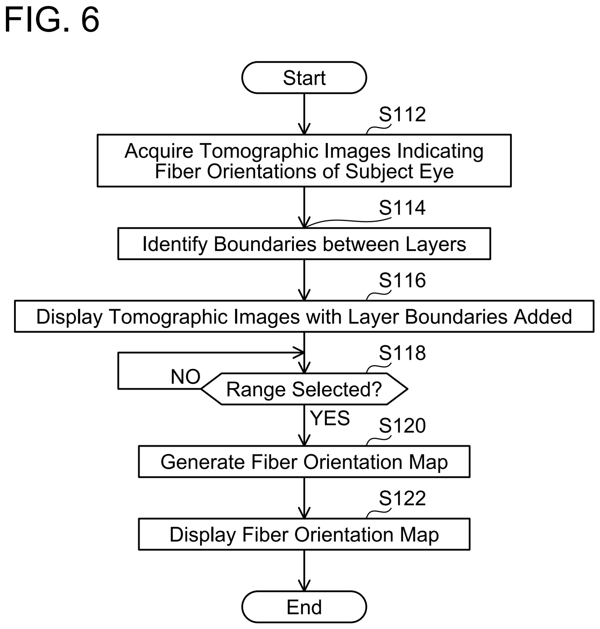

As illustrated in FIG. 6, the processor 202 first acquires tomographic images indicating fiber orientations in the retina of the subject eye 500 (S112). In the present embodiment, the tomographic images indicating fiber orientations are generated using the four interference signals HH, HV, VH, and VV. The tomographic images indicating fiber orientations can be generated by a known method. For example, the tomographic images indicating fiber orientations can be obtained by calculating the birefringence axis, thereby determining the directions of birefringence and visualizing the fiber orientations within the subject eye 500.

Then, the processor 202 identifies boundaries between layers within the subject eye 500 in each tomographic image (S114) and displays the tomographic image with the layer boundaries superimposed thereon on on the monitor 120 (S116). The processor 202 then waits for the user to select a depth range (S118). Since steps S114 to S118 are substantially the same as steps S14 to S18 of the first embodiment, detailed description thereof is omitted.

Once a depth range is selected (YES in S118), the processor 202 generates a fiber orientation map for the depth range selected in S118 (S120). Specifically, the processor 202 generates an en-face image using only the tomographic images (tomographic information) within the depth range selected in S118. As a result, a fiber orientation map for the depth range selected in step S118 is generated. In the present embodiment as well, the fiber orientation map is generated using the tomographic information only from the layer(s) selected by the user. The information on birefringence (the fiber orientation map) only for the desired range is displayed, thereby reducing unnecessary information and improving visibility of the image.

For example, as a result of the user selecting a range r1 including the nerve fiber layer (NFL) and Henle's fiber layer as illustrated in FIG. 7(a), a fiber orientation map indicating only fiber orientations in the nerve fiber layer (NFL) and Henle's fiber layer is displayed as illustrated in FIG. 7(b). Further, as a result of the user selecting a range r2 including the sclera as illustrated in FIG. 8(a), a fiber orientation map indicating only fiber orientations in the sclera is displayed as illustrated in FIG. 8(b). As illustrated in FIGS. 7(b) and 8(b), fiber orientations differ among the layers in the retina of the subject eye 500. If a fiber orientation map for the entire retina of the subject eye 500 is generated, information from multiple layers having different fiber orientations is compressed into an en-face image, making it difficult to recognize fiber orientations. By generating a fiber orientation map only for a depth range selected by the user, fiber orientations in that range can be easily recognized. Recognizing fiber orientations in a particular range (layer) helps to understand how the fiber orientations affect the shape of the subject eye 500. For example, comparing changes in fiber orientations before and after elongation or deformation of an eyeball due to high myopia or pathologic myopia helps to understand the disease of the subject eye 500 and consider treatment strategies.

### (Third Embodiment)

In the second embodiment described above, the processor 202 generates a fiber orientation map for a desired depth range of the subject eye 500, however, the processor 202 may display a fiber orientation map for a desired depth range with lines indicating fiber orientations (directions of the birefringence axes) (hereinafter referred to as streamlines) superimposed thereon.

In the present embodiment, the processor 202 first executes the same sequence as the sequence from steps S112 to S118 of second embodiment. Then, the processor 202 generates a fiber orientation map in the same manner as step S120 of the second embodiment and also generates streamlines. Generally, algorithms or software for drawing streamlines are readily available. For example, streamlines of birefringence axial directions can be drawn by using Advanced Plotting Toolkit available from Heliosphere Research LLC, which is provided as an add-on to LabVIEW from National Instruments. Alternatively, open-source software such as Matplotlib may be used.

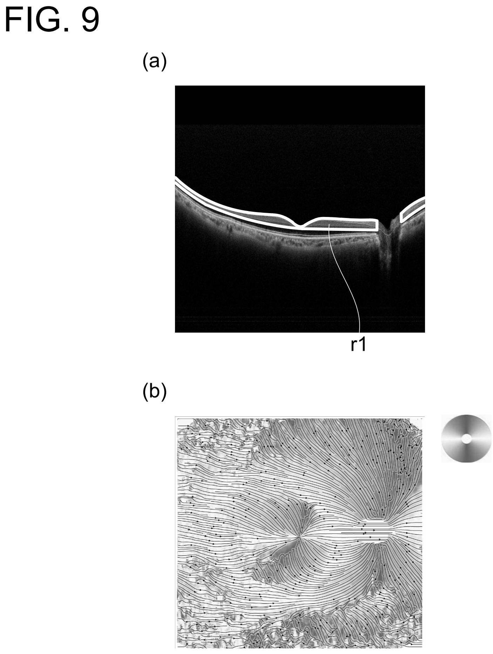

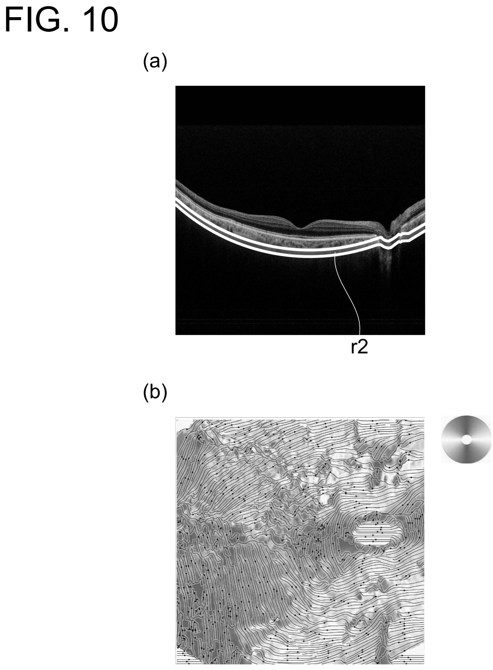

Then, the processor 202 displays the generated fiber orientation map with the streamlines superimposed thereon on the monitor 120. In the present embodiment as well, the fiber orientation map is generated only for layer(s) selected by the user. The information on birefringence only for the desired range (the fiber orientation map) is displayed, thereby reducing unnecessary information and improving visibility of the image. As illustrated in FIGS. 9(b) and 10(b), in the present embodiment, the streamlines are superimposed on the fiber orientation map. A fiber orientation map that indicates axial directions by colors is difficult to interpret intuitively because it requires reading fiber orientations from color differences. By superimposing streamlines on a fiber orientation map, fiber orientations can be easily recognized. In the present embodiment, a fiber orientation map with streamlines superimposed thereon is displayed on the monitor 120, however, the fiber orientation map may be switched between a state of being displayed with the streamlines and a state of being displayed without the streamlines (i.e., only the fiber orientation map is displayed) in response to an instruction input by the user via the input means.

### (Fourth Embodiment)

In the third embodiment described above, the processor 202 generates a fiber orientation map for a desired depth range of the subject eye 500 and displays the generated fiber orientation map with streamlines superimposed thereon, but the processor 202 may superimpose information on regions where the birefringence intensity is greater than a predetermined value (hereinafter referred to as high-birefringence regions) on a fiber orientation map or on streamlines for a desired depth range. Hereinbelow, superimposing information on high-birefringence regions on streamlines is described as an example, however, the information on high-birefringence regions may be superimposed on a fiber orientation map.

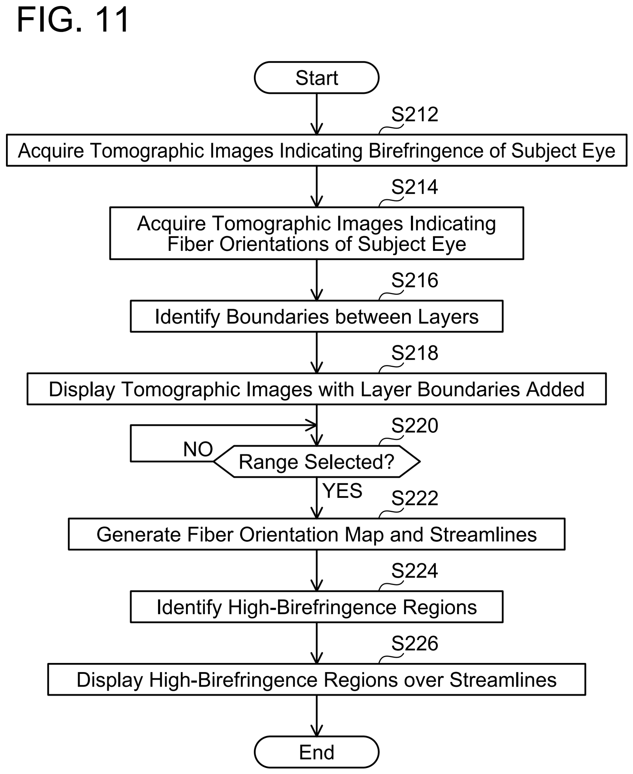

As illustrated in FIG. 11, the processor 202 first acquires tomographic images indicating birefringence of the retina of the subject eye 500 (S212). Since step S212 is the same as step S12 of the first embodiment, detailed description thereof is omitted. Then, the processor 202 acquires tomographic images indicating fiber orientations in the retina of the subject eye 500 (S214). Since step S214 is the same as step S112 of the second embodiment, detailed description thereof is omitted. Step S214 may be performed before step S212. Then, the processor 202 identifies boundaries between layers within the subject eye 500 in each tomographic image (S216) and display the tomographic image with the layer boundaries superimposed thereon on the monitor 120 (S218). The processor 202 then waits for the user to select a depth range (S220). Since the sequence from steps S216 to S220 is substantially the same as the sequence from steps S14 to S18 of the first embodiment, detailed description thereof is omitted.

The processor 202 then uses the tomographic images indicating fiber orientations acquired in step S214 to generate a fiber orientation map and streamlines for the depth range selected in step S220 (S222). Since step S222 is substantially the same as generating a fiber orientation map and streamlines in the third embodiment, detailed description thereof is omitted.

Then, the processor 202 identifies high-birefringence regions in the tomographic images indicating birefringence acquired in step S212 for the depth range selected in step S220 (S224). The predetermined value used for determination on a high-birefringence region is set by the user. That is, the user sets a value that they consider to indicate high birefringence as the predetermined value (i.e., a threshold).

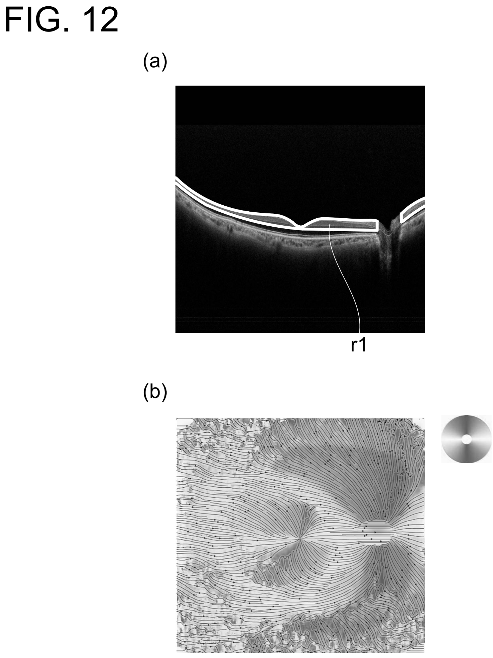

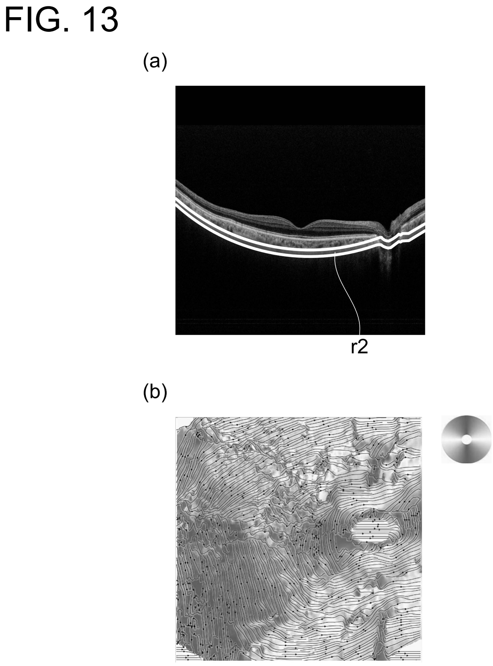

Next, the processor 202 superimposes the high-birefringence regions identified in step S224 onto the streamlines generated in step S222 and displays the result on the monitor 120 (S226). For example, as illustrated in FIGS. 12(b) and 13(b), in the present embodiment, high-birefringence regions are colored (highlighted) in a color different from the color of the streamlines. That is, the color representing high-birefringence regions is superimposed on the streamlines.

Birefringence intensity and fiber orientation are both features related to birefringence of fibrous tissues, but they have clinically different meanings. It is useful to evaluate birefringence intensity and fiber orientation separately, but it is also important to evaluate them comprehensively. If birefringence intensity and fiber orientation are displayed in separate images during comprehensive evaluation, the user has to compare the two images, which is inconvenient. By superimposing high-birefringence regions on streamlines, the user can more easily evaluate birefringence intensity and fiber orientation comprehensively. For example, high birefringence intensity suggests high fiber density, and fiber orientation may have greater clinical significance in high-birefringence regions than in regions with low birefringence (i.e., regions with sparse fiber density). By superimposing high-birefringence regions on streamlines, fiber orientations within the high-birefringence regions can be easily recognized. This allows the user to easily focus on fiber orientations within the high-birefringence regions possibly having greater clinical significance.

In the present embodiment, the entire high-birefringence region is colored in a color different from that of the streamlines, but this need not always be the case. It is sufficient that high-birefringence regions can be visually easily distinguished; for example, streamlines within high-birefringence regions may be colored in a different color from a color of streamlines within the other regions. Alternatively, the width of streamlines within high-birefringence regions may be different from that of streamlines within the other regions, or the brightness of streamlines within high-birefringence regions may be different from that of streamlines within the other regions. The predetermined value may be changed by the user after streamlines and high-birefringence regions have been displayed in a superimposed manner.

### (Fifth Embodiment)

In the first embodiment described above, the processor 202 generates a birefringence intensity map for a desired depth range of the subject eye 500, but the processor 202 may instead generate a thickness map for regions where birefringence intensity is greater than a predetermined value (hereinafter referred to as a high-birefringence region thickness map) for a desired depth range.

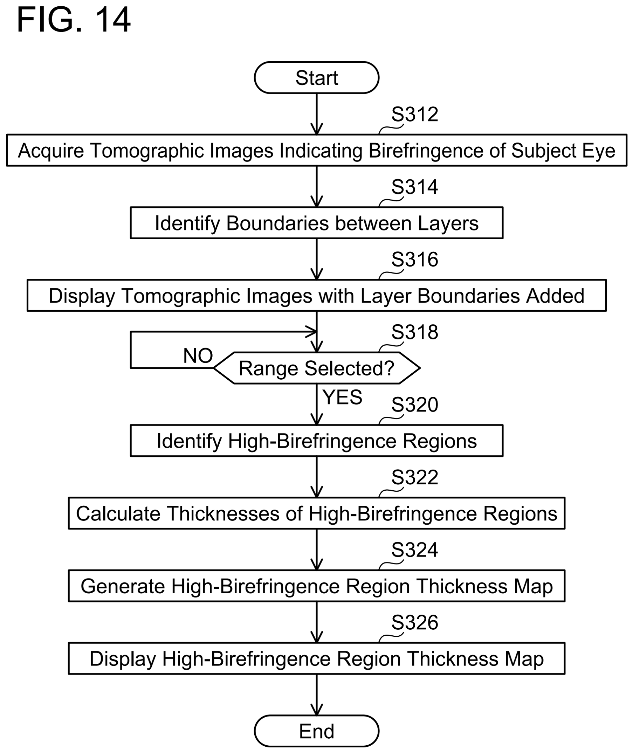

As illustrated in FIG. 14, the processor 202 first acquires tomographic images indicating birefringence of the retina of the subject eye 500 (S312), identifies boundaries between layers within the subject eye 500 in each tomographic image (S314), and displays the tomographic image with the layer boundaries superimposed thereon on the monitor 120 (S316). Then, the processor 202 waits for the user to select a depth range (S318). Since the sequence from steps S312 to S318 is substantially the same as the sequence from steps S12 to S18 of the first embodiment, detailed description thereof is omitted.

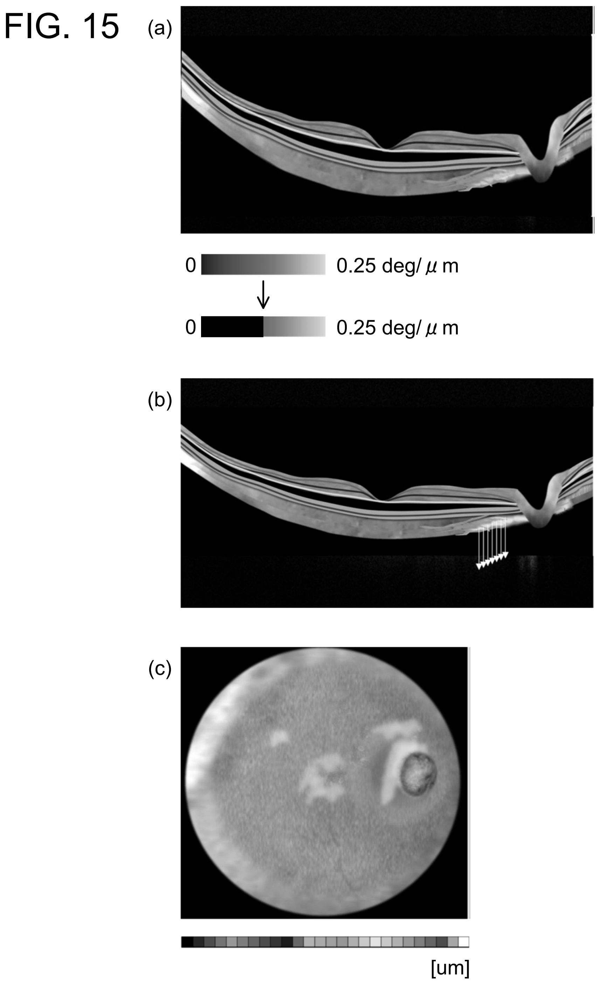

Next, the processor 202 identifies regions where birefringence intensity is greater than a predetermined value (high-birefringence regions) in the tomographic images indicating birefringence acquired in step S312 (S320). The predetermined value is set by the user. That is, the user sets a value that they consider to indicate high birefringence as the predetermined value (i.e., a threshold). For example, assuming that the user selects a depth range deeper than the choroid-sclera interface (CSI) and sets 0.12 deg/µm as the predetermined value (see the ranges below FIG. 15(a)), the processor 202 identifies regions that are deeper than the CSI and have a birefringence intensity of 0.12 deg/µm or greater.

Next, the processor 202 calculates the thicknesses of the high-birefringence regions identified in step S320 for the depth range selected in step S318 (S322). For example, as illustrated in FIG. 15(b), for each tomographic image, the processor 202 calculates the thickness of region where birefringence intensity is 0.12 deg/µm or greater for each A-scan for the depth range selected by the user.

Then, the processor 202 generates a high-birefringence region thickness map from the calculated thicknesses of the high-birefringence regions (S324) and displays the generated high-birefringence region thickness map on the monitor 120 (S326). For example, as illustrated in FIG. 15(c), in the high-birefringence region thickness map, different colors are used according to thickness. In the present embodiment, the high-birefringence region thickness map is displayed only for the depth range selected by the user. Since the high-birefringence region thickness map is generated from information from the depth range selected by the user, information from depth ranges unnecessary to the user is excluded. Therefore, the user can check the thicknesses of high-birefringence regions only for the necessary depth range. Also, since the high-birefringence region thickness map shows only thicknesses of regions where birefringence intensity is greater than the predetermined value, information from layers with low birefringence intensity is excluded. Therefore, the high-birefringence regions can be easily recognized.

Regions with high birefringence intensity suggest high fiber density. If axial elongation progresses and a load is applied to a layer, fiber orientations in the layer may be aligned, thereby increasing fiber density in the layer and resulting in a layer with high fiber density. Alternatively, a layer may intrinsically have high fiber density and thus high resistance to axial elongation. Thus, layers with high fiber density are considered clinically significant regions. For example, the locations of high-birefringence regions can be recognized from a birefringence intensity map (see FIG. 5), but the thicknesses of high-birefringence regions cannot be recognized therefrom. Therefore, to recognize the thicknesses of high-birefringence regions, it was necessary to check individual tomographic images. By displaying a high-birefringence region thickness map, the thicknesses of high-birefringence regions can be recognized at a glance.

### (Sixth Embodiment)

In the fifth embodiment described above, the processor 202 generates a high-birefringence region thickness map for a desired depth range of the subject eye 500, but the processor 202 may instead generate a map indicating the number of layers where a birefringence intensity is greater than a predetermined value (hereinafter referred to as a high-birefringence layer count map) for a desired depth range.

In the present embodiment, the processor 202 first executes the same sequence as the sequence from steps S312 to S320 of the fifth embodiment.

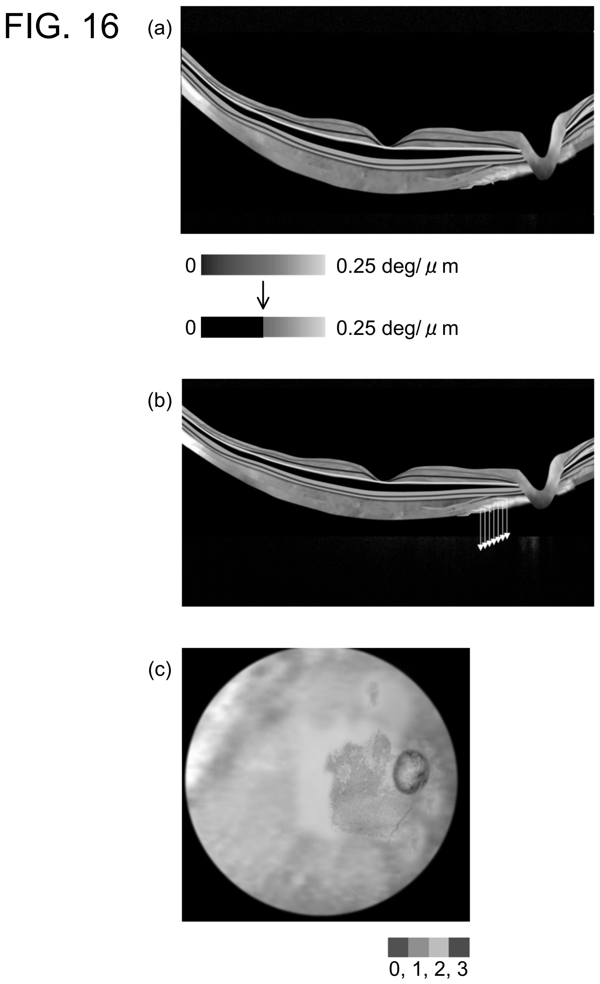

Next, the processor 202 calculates, for the depth range selected in step S318, the number of layers that include the high-birefringence regions identified in step S320. For example, as illustrated in FIG. 16(b), for each tomographic image, the processor 202 calculates the number of layers including regions where a birefringence intensity is 0.12 deg/µm or greater for each A-scan for the depth range selected by the user.

Next, the processor 202 generates a high-birefringence layer count map from the calculated numbers of layers including high-birefringence regions and displays the generated high-birefringence layer count map on the monitor 120. For example, as illustrated in FIG. 16(c), in the high-birefringence layer count map, different colors are used according to the number of layers. In the present embodiment as well, the high-birefringence layer count map is displayed only for the depth range selected by the user. Thus, information unnecessary to the user (information from depth ranges unnecessary to the user and information from layers with low birefringence intensity) is excluded, making it easy to recognize high-birefringence regions. Further, by displaying the high-birefringence layer count map, the number of layers including high-birefringence regions can be recognized at a glance.

### (Seventh Embodiment)

In the second embodiment described above, the processor 202 generates a fiber orientation map for a desired depth range of the subject eye 500, but the processor 202 may instead generate an angle map indicating the sum of relative angles between adjacent layers (hereinafter referred to as a relative angle map) for a desired depth range.

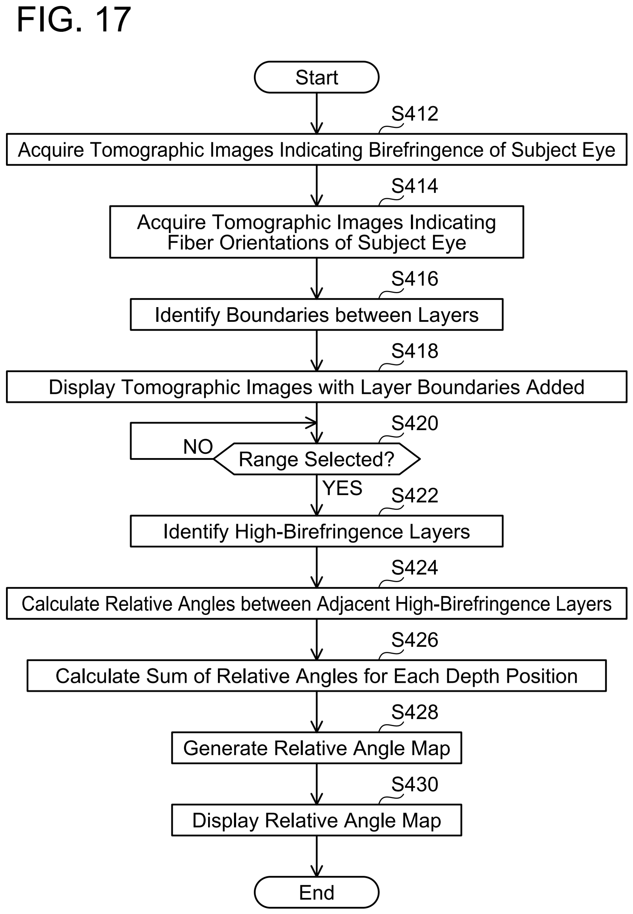

As illustrated in FIG. 17, the processor 202 first acquires tomographic images indicating birefringence of the retina of the subject eye 500 (S412) and acquires tomographic images indicating fiber orientations in the retina of the subject eye 500 (S414). Next, the processor 202 identifies boundaries between layers within the subject eye 500 in each tomographic image (S416) and displays the tomographic image with the layer boundaries superimposed thereon on the monitor 120 (S418). Then, the processor 202 waits for the user to select a depth range (S420). Since the sequence from steps S412 to S420 is substantially the same as the sequence from steps S212 to S220 of the fourth embodiment, detailed description thereof is omitted.

Next, the processor 202 uses the tomographic images indicating birefringence acquired in step S412 to identify layers where a birefringence intensity is greater than a predetermined value (high-birefringence layers) for the depth range selected in step S420 (S422). Specifically, the processor 202 identifies regions where a birefringence intensity is greater than the predetermined value (high-birefringence regions) for the depth range selected in step S420 in each tomographic image indicating birefringence acquired in step S412. Then, the processor 202 identifies the high-birefringence regions located at different positions in the depth direction as different high-birefringence layers. Therefore, the high-birefringence layers are different from the tissue layers identified in step S416, and multiple high-birefringence layers may exist within a single tissue.

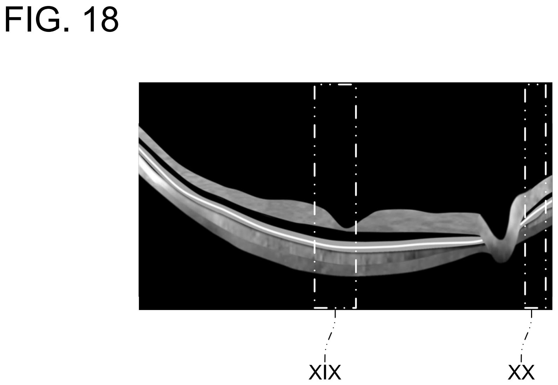

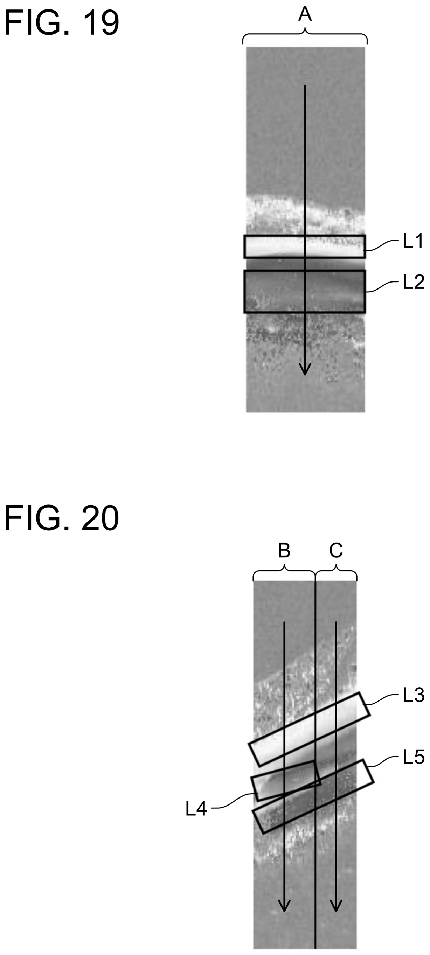

For example, FIG. 18 is a tomographic image indicating birefringence. FIG. 19 is a tomographic image indicating fiber orientations in a relevant area XIX in FIG. 18, and FIG. 20 is a tomographic image indicating fiber orientations in a relevant area XX in FIG. 18. In the relevant area XIX of FIG. 19 (hereinafter referred to as region A), two high-birefringence regions are present at different depth positions. The processor 202 identifies these two high-birefringence regions as high-birefringence layers L1 and L2. In the relevant area XX of FIG. 18 (which is illustrated in FIG. 20), three high-birefringence regions are present at different depth positions. The processor 202 identifies these three high-birefringence regions as high-birefringence layers L3, L4, and L5. The high-birefringence layers L3 and L5 extend across FIG. 20 in left-right direction, whereas the high-birefringence layer L4 is located only on the left-side portion of FIG. 20. That is, in the left-side portion (hereinafter referred to as a region B) of the relevant area XX (which is illustrated in FIG. 20), the three high-birefringence layers L3, L4, L5 are present at different depth positions, while in the right-side portion (hereinafter referred to as a region C) of the relevant area XX, the two high-birefringence layers L3, L5 are present at different depth positions.

Then, the processor 202 calculates relative angles between high-birefringence layers adjacent to each other in the depth direction (S424). Specifically, the processor 202 identifies a fiber orientation (angle) in each high-birefringence layer identified in step S422 and calculates the difference between the fiber orientations (angles) in the high-birefringence layers adjacent to each other in the depth direction for each A scan. Thereby, relative angles are calculated. The fiber orientation in each high-birefringence layer is identified using the tomographic images indicating fiber orientations acquired in step S414. If a single high-birefringence layer has different fiber orientations, the average of the fiber orientations in that high-birefringence layer is calculated and used as the fiber orientation in the high-birefringence layer. Alternatively, if a single high-birefringence layer has different fiber orientations, the fiber orientation at the center position in that high-birefringence layer may be used as the fiber orientation in the high-birefringence layer.

For example, as illustrated in FIG. 19, in the region A, the two high-birefringence layers L1, L2 are identified in step S422. The fiber orientation in the high-birefringence layer L1 is 0 degrees, and the fiber orientation in the high-birefringence layer L2 is 45 degrees. Therefore, the processor 202 calculates a relative angle between the adjacent high-birefringence layers L1 and L2 in the region A as 45 degrees (45 degrees - 0 degrees). Further, as illustrated in FIG. 20, in the region B, the three high-birefringence layers L3, L4, L5 are identified in step S422. The fiber orientation in the high-birefringence layer L3 is 0 degrees, the fiber orientation in the high-birefringence layer L4 is 45 degrees, and the fiber orientation in the high-birefringence layer L5 is 90 degrees. Therefore, the processor 202 calculates a relative angle between the adjacent high-birefringence layers L3 and L4 in the region B as 45 degrees (45 degrees - 0 degrees) and also calculates a relative angle between the adjacent high-birefringence layers L4 and L5 in the region B as 45 degrees (90 degrees - 45 degrees). Further, in the region C, the two high-birefringence layers L3, L5 are identified in step S422. Therefore, the processor 202 calculates a relative angle between the adjacent high-birefringence layers L3 and L5 in the region C as 90 degrees (90 degreed - 0 degrees).

Next, the processor 202 calculates the sum of the relative angles for each depth position (S426). For example, as illustrated in FIG. 19, in the region A, the relative angle calculated in step S424 for each depth position includes the single relative angle of 45 degrees between the high-birefringence layers L1 and L2, so the sum of relative angles is 45 degrees. As illustrated in FIG. 20, in the region B, the relative angles calculated in step S424 for each depth position include two relative angles, namely 45 degrees between the high-birefringence layers L3 and L4 and 45 degrees between the high-birefringence layers L4 and L5, so the sum of relative angles is 90 degrees (45 degreed + 45 degrees). In the region C, the relative angles calculated in step S424 for each depth position includes a single relative angle of 90 degrees between the high-birefringence layers L3 and L5, so the sum of relative angles is 90 degrees.

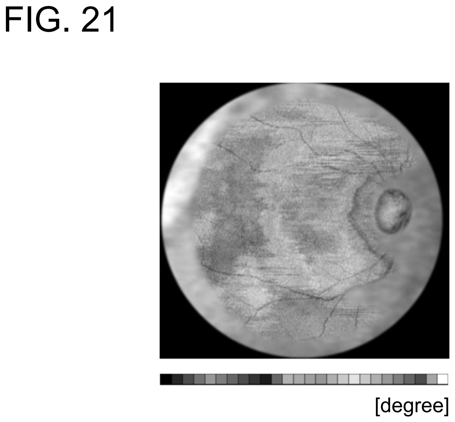

Next, the processor 202 generates a relative-angle map from the sums of relative angles calculated in step S426 (S428) and displays the generated relative-angle map on the monitor 120 (S430). For example, as illustrated in FIG. 21, in the relative-angle map, different colors are used according to the sum of relative angles (degrees).

In the present embodiment as well, the relative-angle map is displayed only for the depth range selected by the user. Therefore, information unnecessary to the user (information from depth ranges unnecessary to the user and information from layers with low birefringence intensity) is excluded, making it easy to recognize the relative angles between the high-birefringence regions. When multiple high-birefringence regions are present at different depth positions, whether their fiber orientations are the same or different may have different clinical implications, for example, regarding stress tolerance to axial elongation. By displaying the relative angle map, the relative angles can be easily recognized.

Specific examples of the disclosure herein have been described in detail, however, these are mere exemplary indications and thus do not limit the scope of the claims. The art described in the claims includes modifications and variations of the specific examples presented above. Technical features described in the description and the drawings may technically be useful alone or in various combinations, and are not limited to the combinations as originally claimed. Further, the purpose of the examples illustrated by the present description or drawings is to satisfy multiple objectives simultaneously, and satisfying any one of those objectives gives technical utility to the present disclosure.

## Claims

1. A polarization-sensitive optical coherence tomographic device comprising:
an image capturing unit configured to capture a tomographic image of a subject eye;
a range specifying means for specifying a depth range of the tomographic image; and
a processor,
wherein
the tomographic image comprises a first tomographic image captured by irradiating the subject eye with first polarized light, and a second tomographic image captured by irradiating the subject eye with second polarized light that has a polarization direction different from that of the first polarized light, and,
the processor is configured to perform an image generation process for generating at least one of an image indicating birefringence and an image indicating a state of fibers for the depth range of the tomographic image specified by the range specifying means based on the first tomographic image and the second tomographic image.

2. The optical coherence tomographic device according to claim 1, further comprising a display unit configured to display the image indicating the state of fibers,
wherein the display unit is configured to display lines indicating fiber directions together with the image indicating the state of fibers such that the lines indicating fiber directions are superimposed on corresponding portions of the image indicating the state of fibers.

3. The optical coherence tomographic device according to claim 2, wherein
the display unit is further configured to display an image indicating a region where a birefringence intensity is greater than a predetermined value so as to be superimposed on a corresponding portion of the image indicating the state of fibers.

4. The optical coherence tomographic device according to claim 1, further comprising a display unit configured to display the image indicating birefringence,
wherein the display unit is configured to display a thickness map indicating thicknesses of regions where birefringence is greater than a predetermined value.

5. The optical coherence tomographic device according to claim 1, further comprising a display unit configured to display the image indicating birefringence,
wherein the processor is configured to further perform:
a layer segmentation process for segmenting layers of the subject eye; and
a determination process for determining, for each of the layers segmented in the layer segmentation process, whether birefringence is greater than a predetermined value or not, and
the display unit is configured to display a layer-count map indicating a number of layers determined to have birefringence greater than the predetermined value in the determination process.

6. The optical coherence tomographic device according to claim 1,
wherein the processor is configured to further perform:
a layer segmentation process for segmenting layers of the subject eye;
a first calculation process for calculating, for each of the layers segmented in the layer segmentation process, a relative angle between the layer and an adjacent layer; and
a second calculation process for calculating a sum of the relative angles calculated in the first calculation process for the depth range of the tomographic image specified by the range specifying means, and
the optical coherence tomographic device further comprises a display unit configured to display an angle map indicating the sum of the relative angles calculated in the second calculation process.
